# EUROPEAN PATENT SPECIFICATION

(11) **EP 1 588 669 B1**
(45) Date of publication and mention of the grant of the patent: **29.08.2007**
(21) Application number: 05252477.4
(22) Date of filing: 20.04.2005
(51) Int. Cl.: A61B 17/17, A61B 17/16, A61B 17/15

(54) **Alignment guide**
Ausrichtungsführung
Guide d'alignement

(30) Priority: 20.04.2004 GB 0408793; 03.09.2004 GB 0419640
(43) Date of publication of application: 26.10.2005
(73) Proprietor: Finsbury (Development) Limited, Leatherhead, Surrey KT22 0BA (GB)
(72) Inventor: Tuke, Michael Antony, Guildford Surrey GU1 3TF (GB); Wozencroft, Robert Michael, Epsom Surrey KT19 8SS (GB)
(74) Representative: Smaggasgale, Gillian Helen

(56) References cited:
- DE-A1- 10 013 331
- GB-A- 2 303 713
- US-A- 4 896 663
- US-A- 5 312 409
- US-A- 6 156 069

## Description

The present invention relates to a tool for use in hip resurfacing operations. More particularly, it relates to a head alignment tool.

The efficient functioning of the hip joints is extremely important to the well being and mobility of the human body. Each hip joint is comprised by the upper portion of the upper leg bone (femur) which terminates in an offset bony neck surmounted by a ball-headed portion which rotates within a socket, known as the acetabulum, in the pelvis. Diseases such as rheumatoid- and osteo-arthritis can cause erosion of the cartilage lining of the acetabulum so that the ball of the femur and the hip bone rub together causing pain and further erosion. Bone erosion may cause the bones themselves to attempt to compensate for the erosion which may result in the bone being reshaped. This misshapen joint may cause pain and may eventually cease to function altogether.

Operations to replace the hip joint with an artificial implant are well-known and widely practiced. Generally, the hip prosthesis will be formed of two components, namely: an acetabular, or socket, component which lines the acetabulum; and a femoral, or stem, component which replaces the femoral head. During the surgical procedure for implanting the hip prosthesis the cartilage is removed from the acetabulum using a reamer such that it will fit the outer surface of the acetabular component of the hip prosthesis. The acetabular component can then be inserted into place. In some arrangements, the acetabular component may simply be held in place by a tight fit with the bone. However, in other arrangements, additional fixing means such as screws or bone cement may be used. The use of additional fixing means help to provide stability in the early stages after the prosthesis has been inserted. In some modem prosthesis, the acetabular component may be coated on its external surface with a bone growth promoting substance which will assist the bone to grow and thereby assist the holding of the acetabular component in place. The bone femoral head will be removed and the femur hollowed using reamers and rasps to accept the prosthesis. The stem portion will then be inserted into the femur.

In some cases, a femoral component of the kind described above may be replaced with components for use in femoral head resurfacing or for use in thrust plate technology.

Although the prosthesis being inserted when the head is being replaced or resurfaced or in thrust plate arrangements is relatively small, the requirement for the surgeon to obtain the necessary access to the hip joint means that it is necessary to make a large incision on one side of the hip. In one technique, a straight incision is made through the skin on the posterior edge of the greater trochanter. In some techniques this incision may be made when the hip is flexed to 45°. By known techniques, the muscles and tendons are parted and held by various retractors such that they do not interfere with the surgeons access to the hip joint. The hip is then dislocated to provide access to the head of the femur.

It will be acknowledged that it is essential that the replacement surface for the head of the femur should be precisely located in both angular and translation positions of the axis of the femoral neck of the implant. To assist this, in some techniques, the surgeon inserts a pin in the lateral femur. The desired position of the pin will be known from pre-operative analysis of the x-rays. The surgeon will measure the desired distance down the femur from the tip of the greater trochanter and the alignment pin is inserted through the vastus lateralis fibres. The alignment pin is inserted in a transverse direction into the mid-lateral cortex and directed upwardly towards the femoral head. The pin is left protruding so that an alignment guide can be hooked over the alignment pin. Suitable alignment guides include those known as the McMinn Alignment Guide available from Midland Medical Technologies Ltd.

Prior art alignment guides of the kind described above generally comprise a hook or aperture which is placed over the alignment pin thus providing a good angular position for the axis of the implant in valgus, varus and ante-version of the neck. The guide will then be adjusted such that a cannulated rod is located such that the aperture therein is directed down the mid-lateral axis of the femoral neck. A stylus having been set to the desired femoral component size is positioned such that it can be passed around the femoral neck. When the stylus can be passed around the femoral neck, the cannulated rod is locked in position. Once the guide is stabilised in this way fine adjustments can be made until the surgeon is happy that the guide is in the required position.

A guide wire can then be inserted though the cannulated rod. This guide wire is then used in the further surgery in which the femoral head is shaped to accept the prosthesis. It will be understood that the alignment guide is an essential tool in the surgical procedure to ensure that the aperture drilled in the femoral head is both central to the femoral neck and at the correct angle of alignment to the femoral neck and that the shaping of the femoral head is accurate for the chosen head size.

It will therefore be understood that it is very important that the alignment guide is positioned correctly. Failure to do so may have the disastrous effect of allowing the machining of the cylinder of the head during the shaping procedure to "notch" into the neck of the femur. This will predispose the bone to early failure on load bearing.

Whilst the prior art alignment tools are particularly suitable for their function and have reached a high level of acceptance among surgeons, there is now a move towards a less invasive surgery in which the required incision should be as small as possible and the amount of interaction with healthy tissue is minimised. It is therefore desirable to consider carrying out femoral head replacement or resurfacing without the need to insert the alignment pin. Thus it is desirable that all of the surgical procedure takes place at the femoral head. There is therefore a requirement for an alignment guide which can function without interaction with an alignment pin.

Other guides are known which are, in use, located on the femoral neck itself. These are used in a similar manner to those described above and may involve some adjustment by the surgeon before he selects the best position.

In addition, it is desirable that the overall function and safety of the alignment guide be improved. It is further desirable that the alignment guide facilitates the accuracy and ease of use of the instruments that work from the neck.

US6156069, US4896663 and US5312409 all describe guides which interact with the femoral head.

Thus according to the present invention there is provided an alignment guide for use in femoral head surgery comprising:
a support arm;
a cannulated rod supported by, and adjustable with respect to, the support arm; and
two jaws, a superior jaw and an inferior jaw, each jaw having a proximal end connected to the support and a distal end for clamping to the neck of the femur in use; characterised in that said jaws are movable from a first open position to a second clamping position; and the jaws remain parallel as they are moved from the first open position to the second clamping position.

The alignment guide is configured such that in use the jaws in the first open position may be passed over the head of the femur and in the second clamping position will clamp against the neck of the femur.

The two jaws will preferably each be movable by the operation of a screw means. Whilst each jaw may have a dedicated screw means, in a preferred arrangement the jaws will be mutually connected at their proximal ends via a screw member having two oppositely threaded ends, each threaded end being associated with a jaw such that when the screw is rotated in one direction the jaws will move towards the center of the screw to the clamped position and when it is rotated in the other direction the jaws move apart to the open position.

The screw means having two oppositely threaded ends will be connected to the support member by any suitable arrangement. In one arrangement where the screw means is the screw member having oppositely threaded ends, the center portion of the screw member, which may be unthreaded, will pass through a receiving portion of the support member.

The screw means will preferably include a head to facilitate the operation of the screw means by the operator.

To improve stability of the tool, the jaws may be connected to the support member by pivot arms.

The jaws may be curved along at least a part of their length such that in use they can extend around the head of the femur and their distal ends can be clamped to the neck of the femur. Alternatively a portion of the length will be substantially straight and in this arrangement, a portion, towards the end of the jaws, remote from the support member will be angled to allow the distal ends to clamp to the neck of the femur.

The tool will preferably be configured such that one jaw is particularly suitable for clamping to the inferior part of the neck (i.e. the underside) and can therefore be referred to as the inferior jaw, and the other is particularly suitable for clamping to the superior part of the neck (i.e. the topside) and can therefore be referred to as the superior jaw.

The two jaws may be of the same or different configurations. Biting elements may be located on one or both jaws to improve the clamping of the jaw with the neck of the femur.

The biting element on the superior jaw may be of any suitable arrangement but is preferably a T-bar located at or near the distal end of the jaw. In use the T-bar will come into contact with the superior part of the neck. The T-bar is preferably cylindrical.

Whilst the jaws may each be of any suitable length, the inferior jaw may be longer than the superior jaw. In one arrangement, the inferior jaw may extend in length over the superior jaw to provide a flag to assist the surgeon to visually ensure that the tool is in the required position. This distal portion of the inferior jaw may be substantially straight.

Where a biting element is located on the inferior jaw, it may be of any suitable configuration. In one arrangement it may be a toothed block. The block may have a concave face between the teeth. The block may comprise four teeth, the teeth will preferably be configured and spaced on the block such that in use they interact with the inferior part of the neck of the femur to cause the tool to be angled at the optimum position. Thus the teeth will enable the tool to be clamped at the correct anteversion angle and at the correct angle from the sagittal plane with these angles being fixed by the femur itself. It is generally believed that there is a portion of the inferior femoral neck located from the head/neck junction of the femur to a position about 2 cms from the head/neck junction which is parallel to the optimum angle for the positioning of the stem of the prosthesis and hence this is often used as an alignment reference.

The optimum position of the tool may be achieved with four teeth in a generally square configuration. The teeth are preferably spaced at from about 10 to about 25 mm apart. They are most preferably spaced at about 15 mm.

In the arrangement where the four teeth are provided in a generally square configuration, the length of the inferior jaw, or the positioning of the block on the inferior jaw will be selected such that the teeth will interact with the portion of the femoral neck detailed above.

The correct axis for insertion of the guide wire into the head of the femur is approximately 30 degrees from the sagittal plane and in 20 degrees anteversion. Thus the jaws and biting elements are configured such that in use the cannulated rod will be located such that a guide wire is inserted at the correct angle. In the most preferred arrangement of the present invention the tool will automatically take the correct orientation from the medial neck.

The cannulated rod is adjustable with respect to the support member. In one arrangement, the rod is a sliding fit in the support arm. Once in the required position the cannulated rod will preferably be lockable such that once locked further movement is prevented. The locking means may be of any suitable arrangement. In one arrangement, a locking screw may be used.

The cannulated rod will in use enable the surgeon to position the guide wire. The cannulated rod may have a slot extending along at least a part of the length of the rod to assist in removing the tool from the guide wire once it is in position.

The cannulated rod will preferably have teeth located at the distal end thereof which in use can be driven into the surface of the femoral head. When driven into the head, these teeth help to clamp the alignment tool in position and to stabilise the tool.

The cannulated rod may additionally function as a measuring or gauging device and to assist this the surface of the rod may include measuring indica to assist the surgeon to know how deep they have cut.

An alignment rod support may be included on the support arm which may support one or more alignment rods which in use will provide a visual guide to assist the surgeon to check that the tool is in the correct position.

The or each alignment rod, which may be of any suitable arrangement, may be fitted into the alignment rod support by any suitable arrangement. One or more apertures may be included in the alignment rod support through which a portion of the alignment rod may be passed. The alignment rod may be a guidewire.

The tool of the present invention may additionally include stylus means of the kind known in the prior art.

The correct axis for insertion of the guide wire into the head of the femur is approximately 30 degrees from the sagittal plane and in 20 degrees of anteversion. Thus the tool of the present invention is configured such that in use the cannulated bore will be located such that the guide wire is inserted at the correct angle. The arrangement of the present invention allows the surgeon to visually check that the tool is in the correct orientation.

In femoral head resurfacing techniques, the surgeon will shape the head of the femur to fit within the cavity of the resurfacing prosthesis. This generally involves a number of shaping steps including the removal of the dome of the femoral head by means of a saw. It is important that the saw cut is made in the correct position so that an accurate fit with the prosthesis can be achieved.

The position of the cut to remove the dome of the femoral head can be calculated from the top of the dome of the femoral head. Thus a saw cutting guide may be located on the cannulated rod such that when the rod is in position, the guide will illustrate the correct position for the cut. Separate guides may be provided for each head size of resurfacing head prosthesis.

In an alternative arrangement, a saw cutting guide may be located on at least one of the jaws.

The alignment guide of the present invention may be used in combination with an elongate distal alignment guide which is described in more detail below.

The alignment guide of the present invention may be used in a method of preparing the head of a femur for femoral head resurfacing wherein the method comprises:
exposing the head of a femur;
locating the alignment guide according to the above first aspect on the neck of the femur; and
machining the head of the femur.

During the surgery, a well may be drilled into the head of the femur via the collar or rod. This well may be the definite hole diameter required of approximately 8 mm and drilled to a depth determined by the tube touching the head. A check may be made with a stylus once the tool is removed and cylinder cutters used guided over a peg placed in the well. These cutters are arranged such that the diameter cut will be correct for the head size chosen and will bottom on the top of the cut head such that the teeth of the cutter do not dangerously over-sail the head-neck junction and cause soft tissue damage or neck notching.

Thus the method preferably comprises:
exposing the head of the femur;
locating the alignment guide according to the above first aspect on the neck of the femur;
inserting a drill through the collar and drilling a well into the head of the femur;
removing the drill;
removing the alignment guide;
removing the top of the head of the femur;
inserting a guide rod into the well;
locating a sleeve cutter on guide rod and cutting the head; and
optionally chamfer cutting the head.

The correct axis for insertion of the guide wire into the head of the femur is approximately 30 degrees from the sagittal plane axis of the femur and in anteversion to allow for the natural offset in each position. Thus the tool of the present invention is configured such that in use the cannulated bore will be located such that the guide wire or drill is inserted at the correct angle. The arrangement of the present invention allows the surgeon to place, and to visually check that the tool is in the correct orientation, and position centered on the femoral head-neck junction.

It will be understood that whilst the tool of the present invention offers particular advantages for minimal invasive surgery, it can also be used in conventional surgical techniques.

The tool of the present invention may be used with all sizes of resurfacing head.

The present invention will now be described by way of example with reference to the accompanying figures in which:
- Figure 1: is a perspective view of the tool of the present invention;
- Figure 2: is a side view of the alignment tool of Figure 1 in use;
- Figure 3: is a perspective view of an alternative configuration of the present invention;
- Figure 4: is a side view of the alignment tool of Figure 3 in use;
- Figure 5: is a schematic illustration of the alignment guide of the present invention in combination with an elongate distal alignment guide showing alignment with the femur;
- Figure 6: is a schematic illustration of the alignment guide of the present invention in use in combination with the elongate distal alignment guide located against the middle of the back of the knee;
- Figure 7: is a perspective view of the arrangement of Figure 5; and
- Figure 8: is an enlarged detail of V of Figure 7.

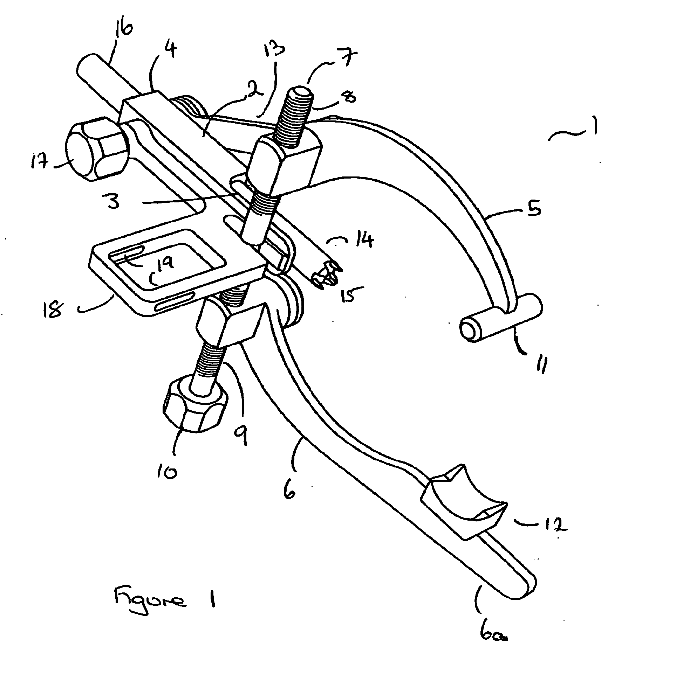

As illustrated in Figure 1, the alignment guide 1 of one embodiment of the present invention comprises a support arm 2 having a distal end 3 and a proximal end 4.

A superior jaw 5 and an inferior jaw 6 are attached to a screw means 7 which comprises a screw member have two oppositely threaded ends 8 and 9 and a head 10. When the head is rotated in one direction the jaws 5 and 6 move inwardly to the clamped position and when rotated in the other direction the jaws 5 and 6 move outwardly to the open position. During movement of the jaws, they remain mutually parallel.

The jaws of this embodiment are curved along at least a part of their length. In the illustrated embodiment, the inferior jaw 6 is longer than the superior jaw 5 to form a flag 6a which is straight.

The biting means on the superior jaw 5 is a cylindrical T-bar 11 which is located at the end of the jaw and perpendicular thereto.

The biting means on the inferior jaw 6 is a toothed block 12 which is located on the straight portion of the jaw.

The jaws are additionally connected to the support means 2 by pivot arms 13.

The cannulated rod 16 is a sliding fit in a sleeve in the support means. A locking screw 17 will enable the user to lock the cannulated rod at the required position. A bore will extend through the rod. Teeth 15 are located on the face of the bore.

A flag holder 18 having apertures 19 through which a flag such as a guide wire may be passed.

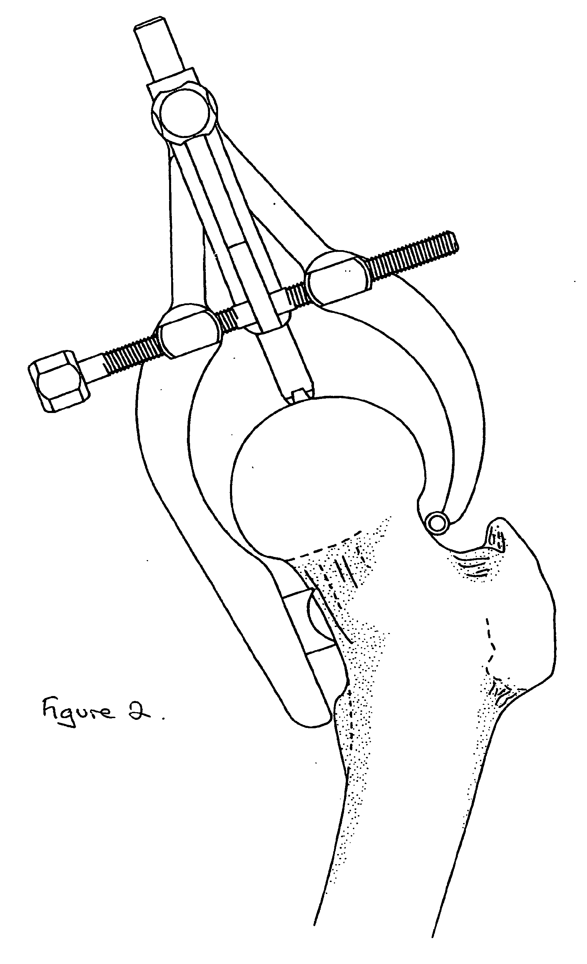

The tool of the first embodiment of the present invention in the clamped position on the neck of a femur is illustrated schematically in Figure 2.

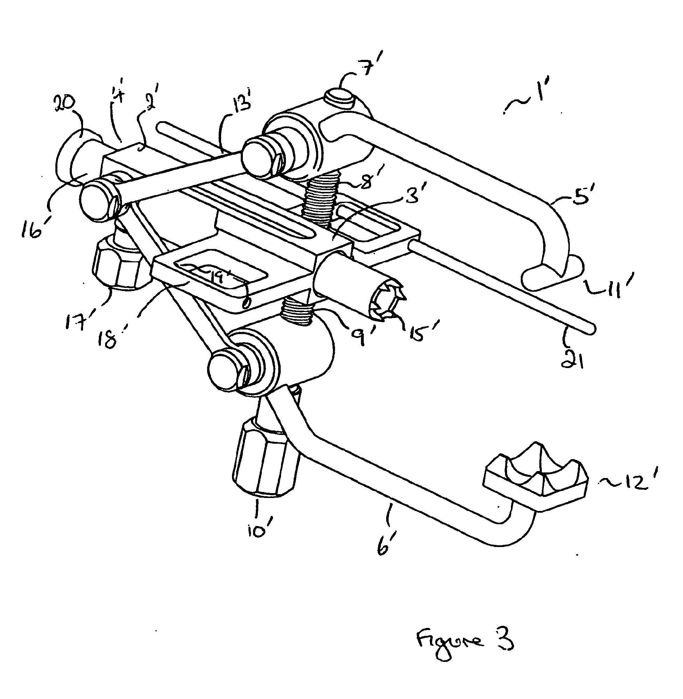

An alternative arrangement is illustrated in Figure 3. The alignment tool 1' of this second embodiment of the present invention comprises a support arm 2' having a distal end 3' and a proximal end 4'.

A superior jaw 5' and a inferior jaw 6' are attached to the screw means 7' which comprises a screw member having two oppositely threaded ends 8' and 9' and a head 10'. When the head is rotated in one direction the jaws 5' and 6' move inwardly to the clamped position and when rotated in the other direction the jaws 5' and 6' move outwardly to the open position. During movement of the jaws they remain mutually parallel.

The jaws of this embodiment are substantially straight along at least a part of their length and are then angled such that in use their ends can interact with the neck of a femur. In the illustrated arrangement, a flag corresponding to 6a in the embodiment of Figure 1 is not present. However, it will be understood that the arrangement of Figure 3 could readily be adjusted to include the flag if required.

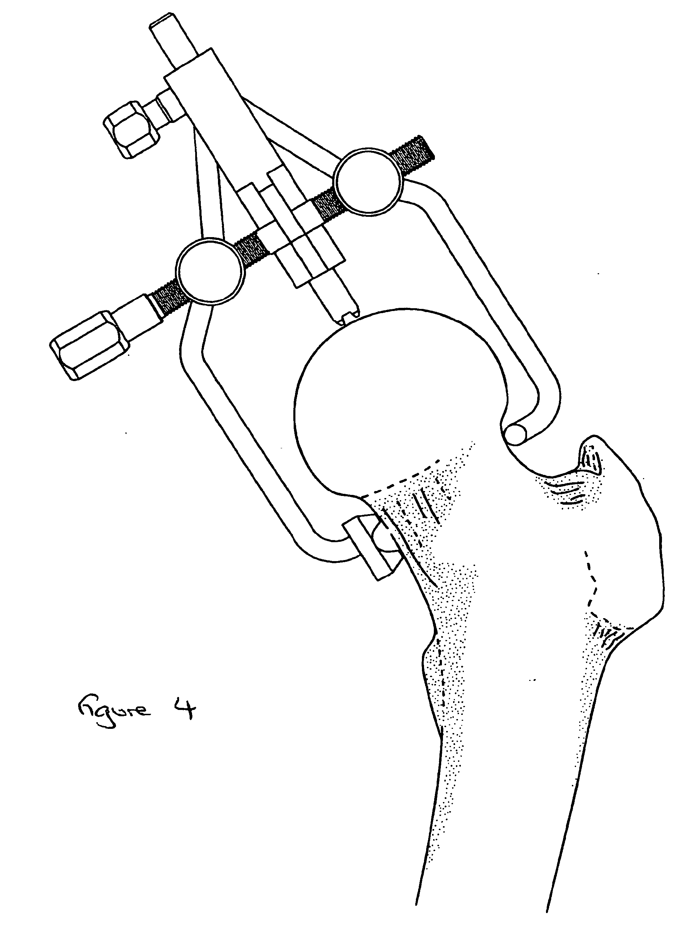

The biting means on the superior jaw 5' is a cylindrical T -bar 11' which is located at the end of the jaw and perpendicular thereto. The biting means on the inferior jaw 6' is a toothed block 12'. The teeth are located about 15 mm apart and as illustrated in Figure 4 interact with the substantially straight portion of the inferior part of the neck of the femur.

The jaws are additionally connected to the support means 2' by pivot arms 13'. The cannulated rod 16' which may include a collar 20' is a sliding fit in a sleeve in the support means. A locking screw 17' will enable the user to lock the cannulated rod at the required position. A ball will extend through the rod. Teeth 15' are located on the face of the ball.

A flag holder 18', which in this embodiment is arranged to hold up to two guide wires is provided. The flag holder 18' has apertures 19' through which a flag such as a guide wire 21' may be passed.

The tool of this embodiment of the present invention in the clamped position on the neck of the femur is illustrated schematically in Figure 4.

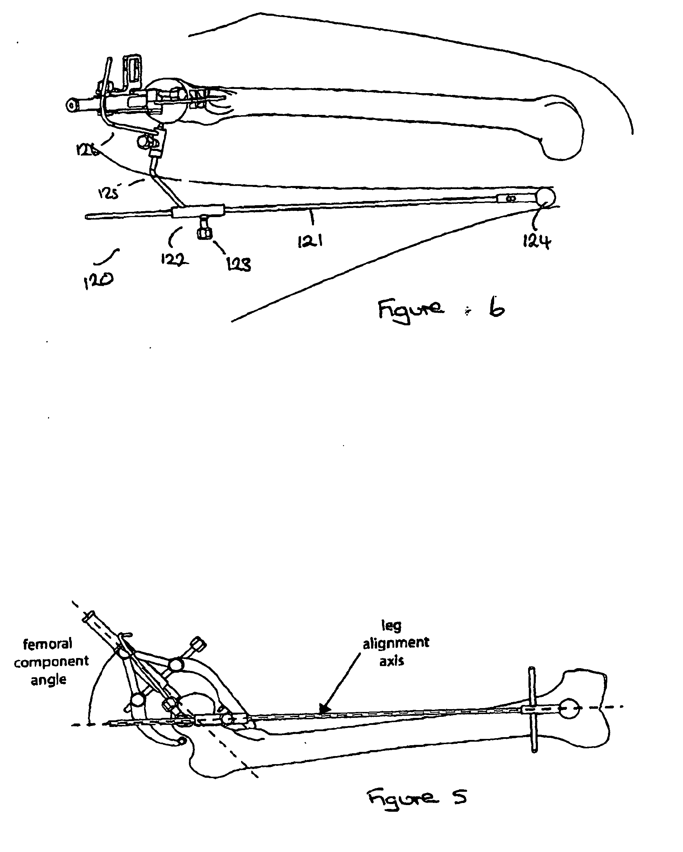

The alignment guide of the present invention may be used in combination with an elongate distal alignment guide 120. The elongate distal alignment guide 120 is illustrated in Figures 5 to 8. The elongate distal alignment guide is used to suggest an optimum femoral component angle for the resurfacing head implant. The alignment guide of the present invention suggests an angle for insertion of the guide wire and ultimately the final implanted femoral resurfacing head prosthesis, relative to the leg alignment axis. The leg alignment axis is a theoretical line between the centre of the femoral head, middle of the knee and middle of the ankle when the person is standing. The axis can be measured easily between the femoral head and knee on a patient in surgery. The position of the axis and the femoral component angle are illustrated in Figure 5.

The elongate distal alignment guide may be attached to the alignment guide of the present invention or it may be a separate component which may be connectable to the alignment guide of the present invention or separate therefrom. Where it is separate, it will touch on the elongate guide of the present invention to measure the current femoral component angle.

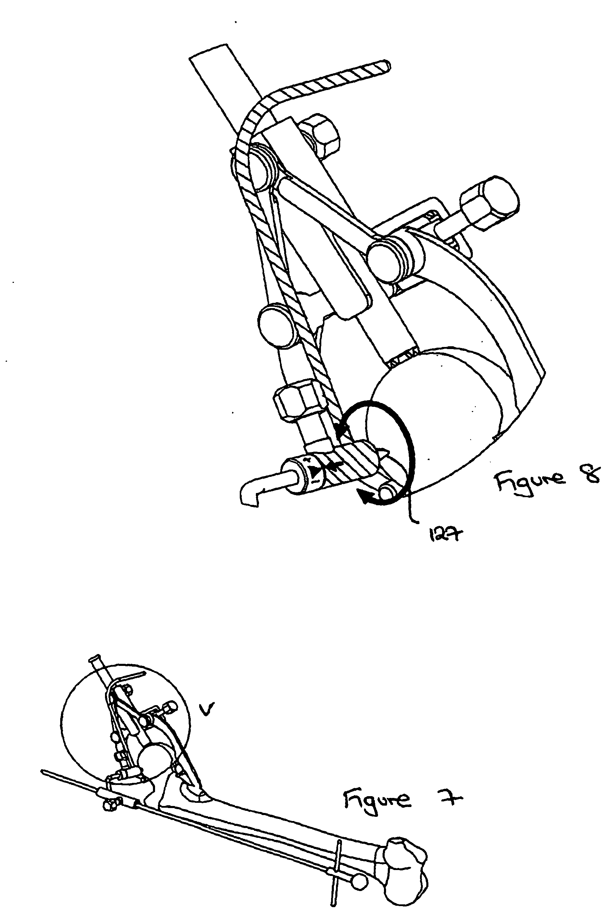

As illustrated in Figure 6, whether the elongate alignment guide is fixed or separate it will generally comprise a long rod 121 slidable in a collar 122 so that its length can be adjusted. A locking nut 123 will be provided to lock the rod at the desired length. A stop 124 may be provided at the end of the rod which in use will come into contact with the back of the patient's knee to protect the skin. Arms 125, 126 extend from the collar 122 and enable the rod to be either fixed to the alignment guide of the present invention or impinge therewith. Arm 126 may be referred to as a probe arm. The angle between the rod 121 and the probe arm 126 may be fixed or it may be adjustable. An adjustable arrangement is illustrated in more detail in Figures 7 and 8. In Figure 8, the "+" sign implies that the alignment guide of the present invention needs to be placed more in valgus and the "-" implies that it needs to be placed in more varus. The area shown hatched in Figure 8 is the area that is free to rotate on the shaft until it touches on the alignment guide of the present invention. In an alternate arrangement, the "+/-" display may be placed with a display showing degrees.

In use, the spiked end 127 should be placed at the centre of the femoral head as this represents the start of the leg alignment axis.

It will be understood that the elongate distal alignment guide described above may be used with any alignment guide of the present invention or any other alignment guide.

## Claims

1. An alignment guide (1) for use in femoral head surgery comprising:
a support arm (2);
a cannulated rod (16) supported by, and adjustable with respect to, the support arm (2), and
two jaws, a superior jaw (5) and a inferior jaw (6), each jaw having a proximal end connected to the support arm (2), and a distal end for clamping to the neck of the femur in use; wherein said jaws are movable from a first open position to a second clamping position; **characterised in that**
the jaws remain parallel as they move from the first open position to the second clamping position.

2. An alignment guide according to Claim I wherein the jaws (5, 6) are movable by a screw means (7).

3. An alignment guide according to Claim 2 wherein the jaws (5, 6) are mutually connected at their proximal ends via a screw member (7) having two oppositely threaded ends (8, 9).

4. An alignment guide according to Claim 3 wherein the center portion of the screw member passes through a receiving portion of the support member.

5. An alignment guide according to Claim 4 wherein the center portion of the screw is unthreaded.

6. An alignment guide according to any one of Claims 3 to 5 wherein the screw means (7) includes a head (10) to facilitate rotation of the screw means.

7. An alignment guide according to any one of Claims 1 to 6 wherein the jaws (5, 6) are connected to the support arm (2) member by pivot arms (13).

8. An alignment guide according to any one of Claims I to 7 wherein the jaws (5, 6) are curved along at least a part of their length.

9. An alignment guide according to any one of Claims 1 to 7 wherein the jaws (5, 6) are straight along at least part of their length.

10. An alignment guide according to any one of Claims 1 to 9 wherein the two jaws (5, 6) are of different configurations.

11. An alignment guide according to any one of Claims 1 to 9 wherein biting elements (11, 12) are located on one or both jaws (5, 6) to improve, in use, the clamping of the jaw with the neck of the femur.

12. An alignment guide according to Claim 11 wherein the biting element on the superior jaw (5) is a T-bar (11) located at or near the distal end of the jaw.

13. An alignment guide according to Claim 1 1 or 12 wherein the biting element on the inferior jaw (6) is a toothed block (12).

14. An alignment guide according to Claim 13 wherein the block (12) has a concave face between the teeth.

15. An alignment guide according to Claim 13 or 14 wherein there are four teeth in a square configuration.

16. An alignment guide according to Claim 15 wherein the teeth are about 15 mm apart.

17. An alignment guide according to any one of Claims I to 16 wherein the cannulated rod (16) is adjustable with respect to the support arm (2).

18. An alignment guide according to Claim 17 wherein the rod (16) is a sliding fit in the support arm (2).

19. An alignment guide according to Claim 17 or 18 wherein the guide additionally includes (17) means for locking the cannulated rod with respect to the support member.

20. An alignment guide according to any one of Claims 1 to 19 wherein the alignment guide additionally includes a saw cutting guide.

21. An alignment guide according to any one of Claims 1 to 20 wherein the alignment guide additionally includes a flag holder (18).

22. A kit comprising the alignment guide of any one of Claims 1 to 21 and an elongate distal alignment guide (20) which by referencing the back of the knee will reference the leg alignment axis.

## Patentansprüche

1. Ausrichtungsführung (1) zur Benutzung bei der Femurkopfchirurgie mit
einem Trägerarm (2),
einem von dem Trägerarm (2) getragenen und zu diesem einstellbaren, röhrenförmigen Stab (16) und
zwei Backen, einer oberen Backe (5) und einer unteren Backe (6), wobei jede Backe ein mit dem Trägerarm (2) verbundenes proximales Ende und ein distales Ende zur Klemmung an dem Femurhals bei Benutzung hat und die genannten Backen aus einer ersten offenen Position in eine zweite klemmende Position beweglich sind, **dadurch gekennzeichnet, daß**
die Backen bei ihrer Bewegung aus der ersten offenen Position in die zweite klemmende Position parallel bleiben.

2. Ausrichtungsführung nach Anspruch 1, bei der die Backen (5,6) durch ein Schraubmittel (7) beweglich sind.

3. Ausrichtungsführung nach Anspruch 2, bei der die Backen (5,6) an ihren proximalen Enden über ein Schraubmittel (7) mit zwei gegenläufigen Gewindeenden (8,9) miteinander verbunden sind.

4. Ausrichtungsführung nach Anspruch 3, bei der der Mittelteil des Schraubelements durch einen Aufnahmeteil des Tragelements hindurchgeht.

5. Ausrichtungsführung nach Anspruch 4, bei der der Mittelteil der Schraube gewindefrei ist.

6. Ausrichtungsführung nach einem der Ansprüche 3 bis 5, bei der das Schraubmittel (7) zur Erleichterung der Drehung des Schraubmittels einen Kopf (10) aufweist.

7. Ausrichtungsführung nach einem der Ansprüche 1 bis 6, bei der die Backen (5,6) durch Dreharme (13) mit dem Trägerarm (2) verbunden sind.

8. Ausrichtungsführung nach einem der Ansprüche 1 bis 7, bei der die Backen (5,6) auf wenigstens einem Teil ihrer Länge gekrümmt sind.

9. Ausrichtungsführung nach einem der Ansprüche 1 bis 7, bei der die Backen (5,6) auf wenigstens einem Teil ihrer Länge gerade sind.

10. Ausrichtungsführung nach einem der Ansprüche 1 bis 9, bei der die zwei Backen (5,6) von unterschiedlicher Ausbildung sind.

11. Ausrichtungsführung nach einem der Ansprüche 1 bis 9, bei der auf einer oder beiden Backen (5,6) Greifelemente (11,12) angeordnet sind, um bei Benutzung die Klemmung der Backe auf dem Femurhals zu verbessern.

12. Ausrichtungsführung nach Anspruch 11, bei der das Greifelement auf der oberen Backe (5) ein an oder in der Nähe des distalen Endes der Backe angeordneter T-Stab (11) ist.

13. Ausrichtungsführung nach Anspruch 11 oder 12, bei der das Greifelement auf der unteren Backe (6) ein gezähnter Block (12) ist.

14. Ausrichtungsführung nach Anspruch 13, bei der der Block (12) zwischen den Zähnen eine konkave Fläche hat.

15. Ausrichtungsführung nach Anspruch 13 oder 14, bei der vier Zähne in einer viereckigen Anordnung vorhanden sind.

16. Ausrichtungsführung nach Anspruch 15, bei der die Zähne etwa 15 mm voneinander getrennt sind.

17. Ausrichtungsführung nach einem der Ansprüche 1 bis 16, bei der der röhrenförmige Stab (16) in Bezug auf den Trägerarm (2) einstellbar ist.

18. Ausrichtungsführung nach Anspruch 17, bei der der Stab (16) mit Gleitsitz in dem Trägerarm (2) ist.

19. Ausrichtungsführung nach Anspruch 17 oder 18, bei der die Führung ferner Mittel (17) zur Verriegelung des röhrenförmigen Stabs in Bezug auf das Trägerelement aufweist.

20. Ausrichtungsführung nach einem der Ansprüche 1 bis 19, bei der die Ausrichtungsführung ferner eine Sägeschnittführung enthält.

21. Ausrichtungsführung nach einem der Ansprüche 1 bis 20, bei der die Ausrichtungsführung ferner einen Kennzeichenhalter (18) enthält.

22. Ausrüstung mit der Ausrichtungsführung eines der Ansprüche 1 bis 21 und einer länglichen distalen Ausrichtungsführung (20), die sich durch Bezugnahme auf die Rückseite des Knies auf die Beinausrichtungsachse bezieht.

## Revendications

1. Guide d'alignement (1) destiné à servir en chirurgie de la tête du fémur, comprenant :
un bras de support (2);
une tige canulée (16) supportée par, et réglable par rapport au bras de support (2), et
deux mors, un mors supérieur (5) et un mords inférieur (6), chaque mors ayant une extrémité proximale reliée au bras de support (2), et une extrémité distale destinée à être serrée sur le col du fémur pendant l'utilisation ; lesdits mors étant mobiles depuis une première position ouverte jusqu'à une seconde position de serrage ; **caractérisé en ce que**
les mors restent parallèles lorsqu'ils passent de la première position ouverte à la seconde position de serrage.

2. Guide d'alignement selon la revendication 1, dans lequel les mors (5, 6) sont mobiles à l'aide d'un moyen formant vis (7).

3. Guide d'alignement selon la revendication 2 dans lequel les mors (5,6) sont reliés l'un à l'autre, au niveau de leurs extrémités proximale, à l'aide d'un élément à vis (7) comportant deux extrémités (8, 9) à filetages opposés.

4. Guide d'alignement selon la revendication 3, dans lequel la partie centrale de l'élément à vis passe par une partie réceptrice de l'élément de support.

5. Guide d'alignement selon la revendication 4, dans lequel la partie centrale de la vis n'est pas filetée.

6. Guide d'alignement selon l'une quelconque des revendications 3 à 5, dans lequel le moyen formant vis (7) comporte une tête (10) pour faciliter la rotation du moyen formant vis.

7. Guide d'alignement selon l'une quelconque des revendications 1 à 6, dans lequel les mors (5, 6) sont reliés au bras de support (2) par des bras pivotants (13).

8. Guide d'alignement selon l'une quelconque des revendications 1 à 7, dans lequel les mors (5, 6) sont incurvés sur au moins une partie de leur longueur.

9. Guide d'alignement selon l'une quelconque des revendications 1 à 7, dans lequel les mors (5, 6) sont rectilignes sur au moins une partie de leur longueur.

10. Guide d'alignement selon l'une quelconque des revendications 1 à 9, dans lequel les deux mors (5, 6) ont des configurations différentes.

11. Guide d'alignement selon l'une quelconque des revendications 1 à 9, dans lequel des éléments d'ancrage (11, 12) sont situés sur un mors ou sur les deux mors (5, 6) afin d'améliorer, pendant l'utilisation, le serrage du mors sur le col du fémur.

12. Guide d'alignement selon la revendication 11, dans lequel l'élément d'ancrage sur le mors supérieur (5) est une barre en T (11) située à ou à proximité de l'extrémité distale du mors.

13. Guide d'alignement selon la revendication 11 ou 12, dans lequel l'élément d'ancrage sur le mors inférieur (6) est un bloc denté (12).

14. Guide d'alignement selon la revendication 13, dans lequel le bloc (12) a une face concave entre les dents.

15. Guide d'alignement selon la revendication 13 ou 14, dans lequel il y a quatre dents disposées en carré.

16. Guide d'alignement selon la revendication 15, dans lequel les dents sont distantes d'environ 15 mm les unes des autres.

17. Guide d'alignement selon l'une quelconque des revendications 1 à 16, dans lequel la tige canulée (16) est réglable par rapport au bras de support (2).

18. Guide d'alignement selon la revendication 17, dans lequel la tige (16) est ajustée de manière coulissante dans le bras de support (2).

19. Guide d'alignement selon la revendication 17 ou 18, le guide comprenant en outre un moyen (17) pour verrouiller la tige canulée par rapport à l'élément de support.

20. Guide d'alignement selon l'une quelconque des revendications 1 à 19, dans lequel le guide d'alignement comporte en outre un guide de sciage.

21. Guide d'alignement selon l'une quelconque des revendications 1 à 20, dans lequel le guide d'alignement comporte en outre un porte-indicateur (18).

22. Kit comprenant le guide d'alignement selon l'une quelconque des revendications 1 à 21 et un guide d'alignement distal allongé (20) qui, en repérant l'arrière du genou, repèrera l'axe d'alignement de la jambe.
